# EUROPEAN PATENT SPECIFICATION

(11) **EP 1 409 666 B1**
(45) Date of publication and mention of the grant of the patent: **29.03.2006**
(21) Application number: 02760263.0
(22) Date of filing: 23.07.2002
(51) Int. Cl.: C12N 15/10, C12Q 1/68, C12N 9/80

(54) **PROCESS FOR PREPARING VARIANT POLYNUCLEOTIDES**
VERFAHREN ZUR HERSTELLUNG VON POLYNUCLEOTIVARIANTEN
PROCEDE DE PREPARATION DE POLYNUCLEOTIDES VARIANTS

(30) Priority: 23.07.2001 EP 01202822; 11.09.2001 EP 01203458
(43) Date of publication of application: 21.04.2004
(73) Proprietor: DSM IP Assets B.V., 6411 TE Heerlen (NL)
(72) Inventor: BOVENBERG, Roelof, Ary, Lans, NL-3062 DA Rotterdam (NL); KERKMAN, Richard, NL-2042 NL Zandvoort (NL)
(74) Representative: van Heuvel, Margaretha
(86) International application number: PCT/EP2002/008222
(87) International publication number: WO 2003/010311

(56) References cited:
- WO-A-98/27230
- WO-A-98/58080
- FR-A- 2 782 323
- KIKUCHI M ET AL: "Novel family shuffling methods for the in vitro evolution of enzymes" GENE, ELSEVIER BIOMEDICAL PRESS. AMSTERDAM, NL, vol. 236, no. 1, 5 August 1999 (1999-08-05), pages 159-167, XP004175459 ISSN: 0378-1119
- BERGER S L ET AL: "PHOENIX MUTAGENESIS: ONE-STEP REASSEMBLY OF MULTIPLY CLEAVED PLASMIDS WITH MIXTURES OF MUTANT AND WILD-TYPE FRAGMENTS" ANALYTICAL BIOCHEMISTRY, ACADEMIC PRESS, SAN DIEGO, CA, US, vol. 214, 1993, pages 571-579, XP002043107 ISSN: 0003-2697
- CRAMERI A ET AL: "DNA SHUFFLING OF A FAMILY OF GENES FROM DIVERSE SPECIES ACCELERATESDIRECTED EVOLUTION" NATURE, MACMILLAN JOURNALS LTD. LONDON, GB, vol. 391, 15 January 1998 (1998-01-15), pages 288-291, XP000775869 ISSN: 0028-0836
- KIKUCHI M ET AL: "An effective family shuffling method using single-stranded DNA" GENE, ELSEVIER BIOMEDICAL PRESS. AMSTERDAM, NL, vol. 243, no. 1-2, February 2000 (2000-02), pages 133-137, XP004187682 ISSN: 0378-1119

## Description

### Background of the Invention

Protein engineering technology includes the creation of novel proteins by targeted modification(s) of known proteins. However, an approach directed to targeted modification is only applicable to proteins or protein families of which the three-dimensional structure of the protein or at least one member protein of the family has been resolved. Furthermore, many attempts to alter the properties of enzymes by this approach have failed because unexpected changes in the structure were introduced. If random mutagenesis is applied to create modified proteins, it appeared that successfully modified proteins often possessed amino acid substitutions in regions that protein modeling could not predict.

Various approaches have been developed to mimic and accelerate nature's recombination strategy to direct the evolution of proteins to more beneficial molecules. Direct evolution is a general term used for methods for random *in vitro* or *in vivo* homologous recombination of pools of homologous polynucleotides. Several formats are described, for instance random fragmentation followed by polymerase-assisted reassembly (WO 9522625), *in vivo* recombination (WO97/07205, WO98/31837) or staggered extension of a population of polynucleotide templates (WO97/07205, WO98/01581). In this way an accumulation of beneficial mutations in one molecule may be accomplished.

FR 2 782 323 discloses an *in vivo* recombination process wherein fragments generated by restriction enzyme digestion are assembled in the presence of an assembly template, whereby the exemplified restriction enzymes are sticky end-generating enzymes.

The method of the present invention advantageously enables the random combination of mutated positions in a rapid, reproducible and highly controllable way. A further advantage of the method of the invention is that the recombination frequency is high and the chance to re-isolate the starting polynucleoti6e is low.

### Detailed Description

The present invention provides a method for the preparation of a variant polynucleotide.
The method according to the invention comprises the steps of:
subjecting separate fractions of a population of polynucleotides to digestions with a restriction enzyme capable of generating blunt-ended fragments,
combining the digests, such that the fragments obtained in the digests serve as each others template in a subsequent reassembly reaction,
applying one or more cycles of denaturation, annealing and reassembly in the presence of a ligase,
optionally amplifying the reassembled polynucleotides,
preparing a library of the resulting variant polynucleotides,
screening said library of variant polynucleotides for a variant polynucleotide with a
desired property.

A variant polynucleotide is defined herein as a polynucleotide differing in at least one position from any one of the members of the population of polynucleotides that forms the starting material for the process according to the invention.

The population of polynucleotides that forms the starting material for the process according to the invention comprises polynucleotide members that display a substantial homology to each other. A substantial homology is defined herein as a homology from 70-100%, preferably from 75-100%, preferably from 80-100%, preferably from 85-100%, more preferably from 90-100%, most preferably from 95-100%. A population of polynucleotides comprising polynucleotide members displaying a substantial homology for instance may be a population of polynucleotides wherein the polynucleotide members are identical polynucleotides, and/or are mutants of a parental polynucleotide and/or are members of a gene family.

A population of mutants derived from a parental polynucleotide may comprise different mutants, each individual mutant in the population differing in at least one position from the parental polynucleotide. A population of different mutants derived from a parental polynucleotide may be obtained by methods known in the art. For instance, the mutants may be obtained by classical random or site-directed mutagenesis techniques. A suitable random mutagenesis technique for instance is the error-prone PCR technique.

The population of mutants may comprise mutants that have been previously screened and selected for a certain desired property.

A population of members of a gene family typically contains different members of a gene family, i.e. polynucleotides displaying a considerable sequence homology, i.e. at least 70%, and having a similar function in an organism. For instance, such polynucleotides may encode related proteins originating from different strains, different species, different genera, different families. An example is the phytase gene family from the genus *Aspergillus,* displaying a homology of at least 90% within the species *Aspergillus niger.*

The starting population of polynucleotides may conveniently be subjected to the process of the invention when being cloned in a vector and/or as isolated fragments. In a situation that the starting population of polynucleotides is obtained by a prior screening and selection process, the vector may conveniently be an expression vector.

According to the method of the invention, separate fractions of the starting population of polynucleotides are subjected to digestion with a restriction enzyme capable of generating blunt-ended fragments.

The restriction enzyme used may be a single enzyme or may be a mixture of two or more enzymes. Preferably, the restriction enzyme(s) and/or the number of separate digestions is (are) chosen in such a way that the mutated positions and/or the regions of heterology as present within the members of the starting population of polynucleotides are located as much as possible on separate fragments. The separate restriction enzyme digests further are performed in such a way that the fragments obtained in the digests can serve as each other's template in a reassembly reaction upon combining the separately digested fractions.

In a preferred embodiment, each separate fraction of the starting population of potynucteotides is digested, with a different restriction enzyme.

According to the invention, the restriction enzyme is capable of generating blunt-ended fragments. By using such a restriction enzyme, the chance of obtaining a substantial amount of the starting polynucleotide(s) after performing the process according to the invention is small.

After inactivating the restriction enzyme(s), the separate digests are combined and the combined digests are subjected to one or more cycles of denaturation, annealing and reassembly in the presence of a ligase.

The number of cycles may be chosen such that a. detectable amount of recombined fragment is obtained. Preferably, 2-100 cycles are performed, more preferably 10-50 cycles, most preferably 20-40 cycles.

The ligase used preferably is a ligase capable of ligating single-strand nicks in a double stranded polynucleotide. Specifically, the ligase is capable of catalysing NAD-dependent ligation of adjacent 3'- hydroxylated and 5'-phosphorylated termini in duplex DNA structures. More preferably, the ligase used is a ligase substantially not capable of ligating blunt-ended polynucleotide fragments, i.e. a ligase with no or a low activity on blunt-ended polynucleotide fragments. Most preferably, the ligase used is a thermostable ligase. An especially preferred ligase is Ampligase (Epicentre).
The products of the ligase-induced reassembly reaction may optionally be amplified by PCR.

A PCR as performed in the method of the invention may be performed following conditions generally known to the person skilled in the art. The conditions typically may depend on the primers and the enzyme used. It may further be an option to perform the PCR under error-prone conditions, i.e. under conditions that reduce the fidelity of nucleotide incorporation, thus randomly introducing additional mutations in the variant polynucleotides obtained by the method of the invention. Error-prone conditions may for instance be provided by independently varying the concentrations of manganese and dGTP in the PCR reaction. Typically, the mutagenesis rate may be raised by increasing the amount of manganese and/or dGTP in the PCR reaction.

The polynucleotide products of the reassembly reaction are cloned in a suitable vector, to enable the preparation of a library of variant polynucleotides. The choice of the vector will depend on the host wherein the library is propagated. Subsequently, the library of variant polynucleotides is screened with a suitable screening method to enable the selection of a variant polynucleotide with a desired property.

The method used for screening the library of variant polynucleotides is not critical for the invention. Typically, the method used will depend on a property of the polynucleotide of interest. If the polynucleotide of interest comprises a gene encoding a polypeptide, a suitable screening method may be directed to directly assay said polypeptide. A suitable screening method may further be directed to assay a primary or secondary metabolite if the polypeptide is an enzyme involved in the production of said primary or secondary metabolite, for instance an enzyme that is part of the biosynthetic pathway of said metabolite. Examples of such metabolites are an amino acid, a vitamin, an antibiotic, a carotenoid.

The method of the invention is suitable for the mutagenesis of any polynucleotide of interest.

In one embodiment of the invention, the polynucleotide of interest comprises a gene encoding a polypeptide. Said polypeptide may for instance be a structural protein, a peptide hormone, a growth factor, an antibody or an enzyme. The polypeptide may be produced intracellularly or may be secreted from the cell into the environment, for instance the culture medium. The polynucleotide may comprise a single gene or may comprise a cluster of genes. Said cluster of genes may comprise genes encoding enzymes involved in the biosynthesis of a particular metabolite and/or genes encoding regulatory factors involved in the regulation of expression of one or more genes involved in production of a particular metabolite.

In another embodiment of the invention, the polynucleotide of interest may be a non-coding polynucleotide, for instance a regulatory region involved in the control of gene expression, on transcriptional and/or translational level.The process of the invention may also be applied to a polynucleotide comprising a gene (cluster) and corresponding regulatory regions.

The present invention further envisages production of a variant polypeptide by expressing a variant polynucleotide produced and selected according to the invention in a suitable host organism and, optionally, recovery of the produced polypeptide.

To this end, the selected polynucleotide is cloned in an expression vector of choice and transformed to a host organism of choice. Transformed host cells are selected from the untransformed background by any suitable means. The transformed cells are grown in a suitable culture medium and may further be screened for expression of the variant polynucleotide. Techniques for the transformation of host cells and for the selection of transformed cells are commonly known to the skilled person.

For production of the variant polypeptide on a larger scale, a transformed cell producing a suitable amount of the variant polypeptide of interest may be cultured under conditions conducive to the production of said polypeptide. Optionally, the polypeptide may be recovered from the culture medium and/or form the host organism. Depending on its further use, recovery of the variant polypeptide may include its formulation in a suitable liquid or solid formulation, and/or its immobilization.

### Brief description of the drawings

Figure 1. Schematic illustration of the BERE recombination technique.
Figure 2. Blunt-end restriction enzyme fragmentation used for the BERE recombination method.
Figure 3. Agarose gel electrophoresis of the reassembly reaction. The arrow indicates DNA bands of the appropriate size (~1kb).
   1: Marker
   2: Reassembly (+ Ampligase), 15 cycles
   3: Reassembly (+ Ampligase), 30 cycles
   4: Starting material (all restriction fragments
Figure 4. Typical results of the conversion activities of a group of mutants from the KKN05 library selected after the first MTP analysis.

### Experimental

### MTP screening

Single colonies of the library to be screened were inoculated in individual wells of microtiter plates (MTP's) filled with SE liquid medium (containing bacto tryptone 10 g/l, bacto yeast 5 g/l and NaCl 5 g/l), supplemented with ampicillin at a final concentration of 100 µg/ml. If required, arabinose inducer was added (final concentration 0.002%). Normal growth conditions were at 37 °C; induced growth conditions were at 28 °C and 280 rpm. 50 µl of the 20-24 hour grown cultures were incubated with D,L-α-methylphenylglycine amide (Femam) at 55 °C in deepwell plates. After 2.5 hours of incubation the amidase activity of the culture broth was measured by measuring the amount of formed L-α-methylphenylglycine. (Femac).

### CFE screening

Cell-free extracts (CFE's) were prepared using a bacterial protein extraction reagent according to the manufacturer's instructions (BPER, Pierce, Rockford, IL USA) and their L-amidase activity was measured.

### Amidase activity assay

Amidase activity was measured as conversion activity from Femam to Femac. Detection occurred by NMR.

### Example 1

### Preparation and screening of an error-prone library of O. anthropi L-amidase

An error-prone PCR was performed on the *Ochrobactrum anthropi* L-amidase gene (see SEQ ID No 1) using the Diversify™ PCR Random Mutagenesis kit from Clontech (Palo Alto, CA USA) according to the manufacturer's instructions. The PCR products were cloned in the Eagl / Hindlll sites of the vector pBAD/Myc-HisC (Invitrogen Corporation, Carlsbad, CA USA) and transformed to *E. coli* Top10F cells (Invitrogen Corporation, Carlsbad, CA USA). Clones were first screened on MTP and CFE's of a subset of clones were further screened (see Experimental). Improved mutants were sequenced to determine the modified position(s). The modified positions of seven improved mutants are indicated hereinafter: V52A, F93V, T143A, T193P, N212D, N98I/L124P, K138R/G234V (see SEQ ID No 2).

### Example 2

### Recombination of improved mutants by BERE recombination

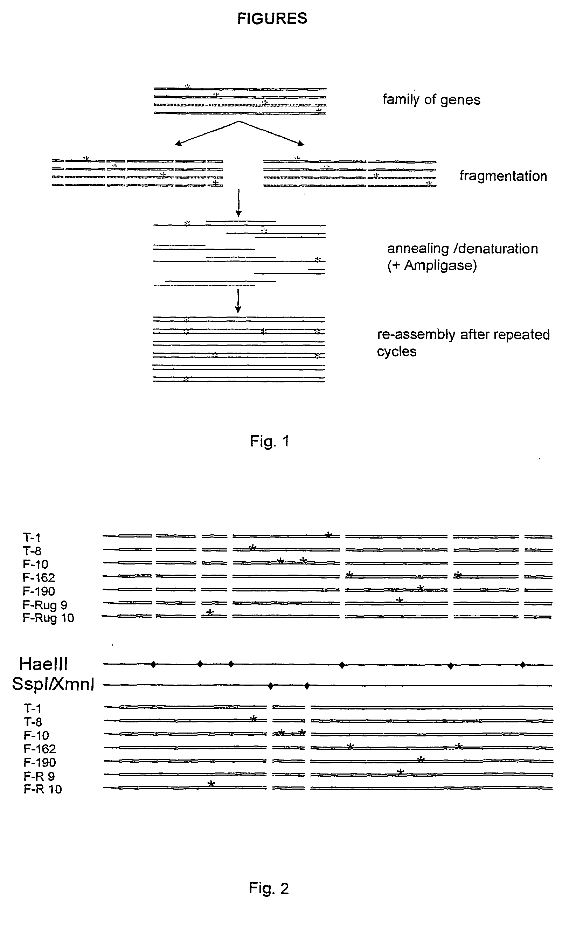

An oultline of the blunt-ended restriction enzyme (BERE) method is given in Figure 1.

DNA of the seven mutant L-amidase genes as described in Example 1 was either digested with Xmnl/Sspl or with Haelll. Two out of the total nine mutations were still located on one fragment after restriction enzyme fragmentation and therefore could not be recombined separately (see Figure 2).

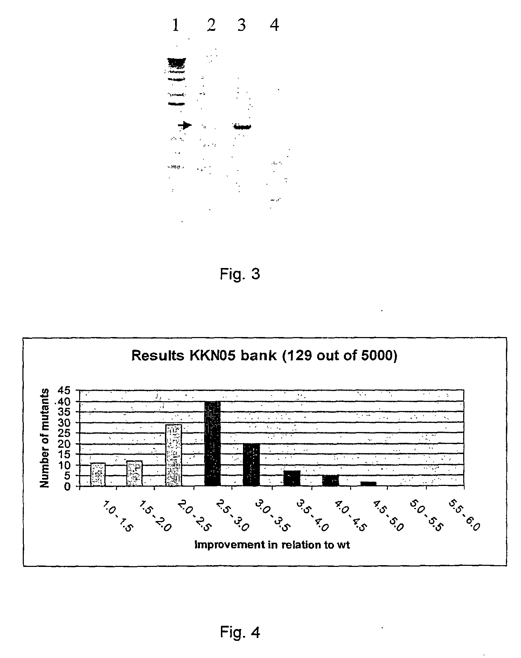

The fragments of both digestions were mixed and used for a reassembly reaction using Ampligase (Epicentre Technologies, Madison, WI USA). As can been seen in Figure 3, already after 15 cycles of denaturation and annealing a DNA of the appropriate size appears.

The DNA product of the Ampligase-induced reassembly reaction was subsequently used as template for an error-prone PCR (EP-PCR).
For this experiment mild EP conditions designed to generate an average of around 1 basepair substitution per gene were used. The DNA products were cloned in the Eagl / Hindlll sites of the pBAD/Myc-HisC vector and transformed to *E. coli* Top10F cells.

Of the resulting KKN05 library 5000 mutants were screened in MTP as described in Experimental.

DNA of the L-amidase genes of 10 randomly picked mutants from the KKN05 library was sequenced. The results of the sequence analysis are presented in Table 1. From the sequence results of the randomly picked mutants a recombination frequency of the mutations of at least 20% and an error prone frequency of 0.6 mutations/gene was calculated.
A large group of mutants selected from the MTP screening was tested in a secondary screening. In this screening, CVE's and different dilutions thereof were analysed for conversion activity by NMR. The conversion/µl was calculated and corrected for the amount of protein. Subsequently the conversion/µl/mg protein of the mutants was compared with conversion/µl/mg protein of the wild type, and the activity improvement was determined.

In Figure 4 the overall results of selected mutants from the KKN05 library are presented.

After one round of BERE recombination, the specific activity of the mutants was substantially increased (Figure 4). The mutant with the highest improvement turned out to be 4-5 times more active than wild type.
Subsequently DNA of the L-amidase genes of the top 10-improved mutants from the KKN05 library was sequenced. The results of the sequence analyses are presented in Table 1.

Five mutants turned out to be unique. Within two mutants additional mutations caused by EP PCR was demonstrated.

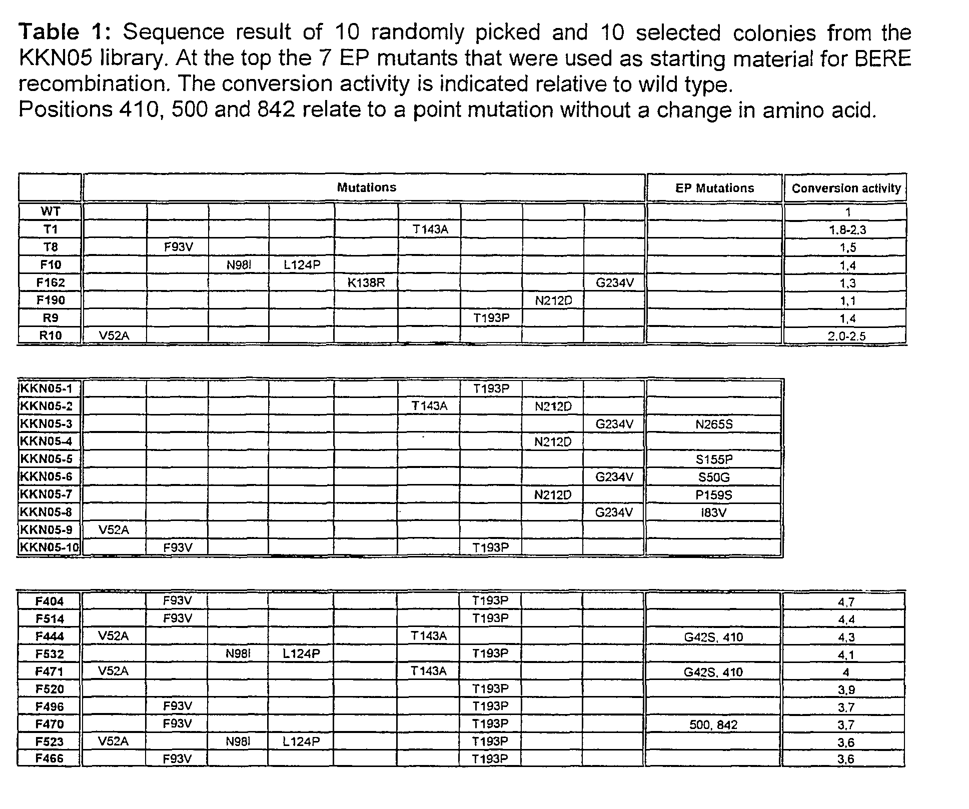

## Claims

1. A process for the preparation of a variant polynucleotide having a desired property, comprising:
subjecting a population of polynucleotides to separate digestions with a restriction enzyme capable of generating blunt-ended fragments;
combining the digests such that the fragments obtained in the digests serve as each other's template in a subsequent reassembly reaction;
applying one or more cycles of denaturation, annealing and reassembly in the presence of a ligase capable of ligating single-strand nicks in a double stranded polynucleotide;
optionally amplifying the reassembled polynucleotides;
preparing a library of the resulting variant polynucleotides;
screening said library of variant polynucleotides for a variant polynucleotide with a desired property.

2. The process of claim 1, wherein the members of the population of polynucleotides display an homology of at least 70%, preferably of at least 75%, preferably of at least 80%, preferably of at least 85%, more preferably of at least 90%, most preferably of at least 95%.

3. The process of claim 1, wherein the population of polynucleotides is selected from the group consisting of a population of different mutants of a parental polynucleotide and a population of different members of a gene family.

4. The process of claim 1, wherein the ligase is substantially not capable of ligating blunt-ended polynucleotide fragments.

5. The process of claim 1, wherein the ligase is a thermostable ligase.

6. The process of claim 1, wherein the amplification of the reassembled polynucleotides is performed under error-prone conditions.

7. The process of claim 1, wherein the polynucleotide comprises one or more gene(s) encoding a polypeptide.

8. The process of claim 8, wherein the polypeptide is involved in the biosynthetic pathway of a primary or secondary metabolite.

9. A process for the preparation of a variant polypeptide comprising the preparation of a variant polynucleotide according to a process according to claims 1 to 8, selecting a variant polynucleotide and expressing this variant polynucleotide into a variant polypeptide.

10. A process for the preparation of a primary or secondary metabolite comprising the preparation of a variant polynucleotide according to a process according to claims 1 to 8, selecting a variant polynucleotide and expressing this variant polynucleotide into a primary or secondary metabolite.

## Patentansprüche

1. Verfahren zur Herstellung einer Polynukleotidvariante mit einer gewünschten Eigenschaft, das die folgenden Schritte umfaßt:
Durchführen von getrennten Restriktionen an einer Population von Polynukleotiden mit einem Restriktionsenzym, das zur Erzeugung von Fragmenten mit glatten Enden fähig ist;
Zusammengeben der Restriktionsprodukte auf solche Art und Weise, daß die bei den Restriktionen erhaltenen Fragmente in einer anschließenden Reassemblierung als Matrizen füreinander dienen;
Durchführung von einem oder mehreren Denaturierungs-, Reassoziations- und Reassemblierungszyklen in Gegenwart einer Ligase, die fähig ist, Einzelstrangbrüche in einen doppelsträngigen Polynukleotid zu ligieren;
gegebenenfalls Amplifizieren der reassemblierten Polynukleotide;
Herstellen einer Bibliothek der erhaltenen Polynukleotidvarianten;
Durchmustern dieser Bibliothek von Polynukleotidvarianten auf eine Polynukleotidvariante mit einer gewünschten Eigenschaft.

2. Verfahren nach Anspruch 1, wobei die Mitglieder der Polynukleotidpopulation mindestens 70%, vorzugsweise mindestens 75%, vorzugsweise mindestens 80%, vorzugsweise mindestens 85%, stärker bevorzugt mindestens 90% und am stärksten bevorzugt mindestens 95% Homologie aufweisen.

3. Verfahren nach Anspruch 1, wobei die Polynukleotidpopulation aus der Gruppe, die aus einer Population von unterschiedlichen Mutanten eines Mutterpolynukleotids und einer Population von unterschiedlichen Mitgliedern einer Genfamilie besteht, stammt.

4. Verfahren nach Anspruch 1, wobei die Ligase im wesentlichen nicht fähig ist, Polynukleotidfragmente mit glatten Enden zu ligieren.

5. Verfahren nach Anspruch 1, wobei es sich bei der Ligase um eine hitzestabile Ligase handelt.

6. Verfahren nach Anspruch 1, wobei die Amplifikation der reassemblierten Polynukleotide unter Error-prone-Bedingungen durchgeführt wird.

7. Verfahren nach Anspruch 1, wobei das Polynukleotid ein oder mehrere Gen(e), das bzw. die für ein Polypeptid codiert/codieren, umfaßt.

8. Verfahren nach Anspruch 8, wobei das Polypeptid an einem Biosyntheseweg eines primären oder sekundären Metaboliten beteiligt ist.

9. Verfahren zur Herstellung einer Polypeptidvariante, das die Herstellung einer Polynukleotidvariante nach einem Verfahren nach den Ansprüchen 1 bis 8, die Auswahl einer Polynukleotidvariante und die Expression dieser Polynukleotidvariante zu einer Polypeptidvariante umfaßt.

10. Verfahren zur Herstellung eines primären oder sekundären Metaboliten, das die Herstellung einer Polynukleotidvariante nach einem Verfahren nach den Ansprüchen 1 bis 8, die Auswahl einer Polynukleotidvariante und die Expression dieser Polynukleotidvariante zu einem primären oder sekundären Metaboliten umfaßt.

## Revendications

1. Procédé pour la préparation d'une variante de polynucléotide présentant une propriété souhaitée, comprenant :
le fait de soumettre une population de polynucléotides à des digestions distinctes avec une enzyme de restriction pouvant générer des fragments à extrémité franche;
la combinaison des produits de digestion de manière à ce que les fragments obtenus dans les produits de digestion servent mutuellement de matrice dans une réaction ultérieure de réassemblage;
l'application d'un ou de plusieurs cycles de dénaturation, annelage et réassemblage en présence d'une ligase pouvant réaliser des ligations de coupures simple brin dans un polynucléotide double brin;
l'amplification facultative des polynucléotides réassemblés;
la préparation d'une banque des variantes obtenues de polynucléotides;
le criblage de ladite banque de variantes de polynucléotides en vue d'obtenir une variante de polynucléotide présentant une propriété souhaitée.

2. Procédé suivant la revendication 1, dans lequel les membres de la population de polynucléotides présentent une homologie d'au moins 70%, de préférence d'au moins 75%, de préférence d'au moins 80%, de préférence d'au moins 85%, de manière plus préférée d'au moins 90%, de manière la plus préférée d'au moins 95%.

3. Procédé suivant la revendication 1, dans lequel la population de polynucléotides est sélectionnée parmi le groupe comprenant une population de différents mutants d'un polynucléotide parent et une population de différents membres d'une famille de gènes.

4. Procédé suivant la revendication 1, dans lequel la ligase est essentiellement incapable de réaliser une ligation de fragments de polynucléotides à extrémités franches.

5. Procédé suivant la revendication 1, dans lequel la ligase est une ligase thermostable.

6. Procédé suivant la revendication 1, dans lequel l'amplification des polynucléotides réassemblés est réalisée dans des conditions générant des erreurs.

7. Procédé suivant la revendication 1, dans lequel le polypeptide comprend un ou plusieurs gène(s) codant pour un polypeptide.

8. Procédé suivant la revendication 8, dans lequel le polypeptide est impliqué dans la voie de biosynthèse d'un métabolite primaire ou secondaire.

9. Procédé pour la préparation d'une variante de polypeptide comprenant la préparation d'une variante de polynucléotide suivant un procédé suivant les revendications 1 à 8, la sélection d'une variante de polynucléotide et l'expression de cette variante de polynucléotide en une variante de polypeptide.

10. Procédé pour la préparation d'un métabolite primaire ou secondaire comprenant la préparation d'une variante de polynucléotide suivant un procédé suivant les revendications 1 à 8, la sélection d'une variante de polynucléotide et l'expression de ladite variante de polynucléotide en un métabolite primaire ou secondaire.


### SEQUENCE LISTING

<110> DSM NV
<120> Process for preparing variant polynucleotides
<130> 20396WO
<160> 2
<170> PatentIn version 3.1
<210> 1
   <211> 945
   <212> DNA
   <213> Ochrobactrum anthropi
<220>
   <221> CDS
   <222> (1)..(945)
   <223>
<400> 1
   atg tgc aat aat tgc cat tac acc att cac ggc cgg cat cat cat ttc      48
   Met Cys Asn Asn Cys His Tyr Thr Ile His Gly Arg His His His Phe
   1               5                   10                  15
   ggc tgg gac aac tcg ttc cag ccg gct gaa acg gtc gcg ccc ggc tcg      96
   Gly Trp Asp Asn Ser Phe Gln Pro Ala Glu Thr Val Ala Pro Gly Ser
               20                  25                  30
   acc ctg aaa ttc gaa tgt ctg gac agc ggc gca ggc cac tat cat cgc     144
   Thr Leu Lys Phe Glu Cys Leu Asp Ser Gly Ala Gly His Tyr His Arg
           35                  40                  45
   ggc agc aca gtc gcc gat gtg tcg acg atg gat ttt tcc aag gtc aat     192
   Gly Ser Thr Val Ala Asp Val Ser Thr Met Asp Phe Ser Lys Val Asn
       50                  55                  60
   ccg gtt acc ggc ccc atc ttc gtc gat gga gcc aaa ccg ggc gat gtc     240
   Pro Val Thr Gly Pro Ile Phe Val Asp Gly Ala Lys Pro Gly Asp Val
   65                  70                  75                  80
   ctg aaa atc acc atc cac cag ttc gag cca tca ggc ttc ggc tgg acg     288
   Leu Lys Ile Thr Ile His Gln Phe Glu Pro Ser Gly Phe Gly Trp Thr
                   85                  90                  95
   gca aat att ccg ggc ttc ggt ctt ctc gcc gac gac ttc aag gaa ccg     336
   Ala Asn Ile Pro Gly Phe Gly Leu Leu Ala Asp Asp Phe Lys Glu Pro
               100                 105                 110
   gcg cta gca ttg tgg aac tac aat ccc aca acg ctg gag cca gca ctc     384
   Ala Leu Ala Leu Trp Asn Tyr Asn Pro Thr Thr Leu Glu Pro Ala Leu
           115                 120                 125
   ttc gga gag cgt gcg cgc gtg ccg ctg aag ccg ttc gcc gga acc atc     432
   Phe Gly Glu Arg Ala Arg Val Pro Leu Lys Pro Phe Ala Gly Thr Ile
       130                 135                 140
   ggc gtc gca ccg gcg gaa aag ggc ctg cat tcg gtc gta cca ccg cgt      480
   Gly Val Ala Pro Ala Glu Lys Gly Leu His Ser Val Val Pro Pro Arg
   145                 150                 155                 160
   cgt gtc ggc ggc aat ctc gac atc cgc gat ctt gca gcc gga acc acg      528
   Arg Val Gly Gly Asn Leu Asp Ile Arg Asp Leu Ala Ala Gly Thr Thr
                   165                 170                 175
   ctt tat ctg ccg atc gaa gtc gaa ggc gct ttg ttc tcc att ggt gat      576
   Leu Tyr Leu Pro Ile Glu Val Glu Gly Ala Leu Phe Ser Ile Gly Asp
               180                 185                 190
   acc cat gcg gca cag ggc gac ggc gaa gtg tgc ggc acc gcc atc gaa      624
   Thr His Ala Ala Gln Gly Asp Gly Glu Val Cys Gly Thr Ala Ile Glu
           195                 200                 205
   agc gcg atg aat gtc gct ctg acg ctg gat ctc atc aag gat acg cca      672
   Ser Ala Met Asn Val Ala Leu Thr Leu Asp Leu Ile Lys Asp Thr Pro
       210                 215                 220
   ctg aag atg ccc cgg ttc acc acg ccg ggg cca gtg acg cgg cac ctc      720
   Leu Lys Met Pro Arg Phe Thr Thr Pro Gly Pro Val Thr Arg His Leu
   225                 230                 235                 240
   gat acc aag ggt tac gaa gtc acc acc ggt atc ggg tcc gat ctg tgg      768
   Asp Thr Lys Gly Tyr Glu Val Thr Thr Gly Ile Gly Ser Asp Leu Trp
                   245                 250                 255
   gaa ggc gcg aaa gcc gcc ctc tcc aac atg atc gac ctt ctt tgc cag      816
   Glu Gly Ala Lys Ala Ala Leu Ser Asn Met Ile Asp Leu Leu Cys Gln
               260                 265                 270
   acg cag aac ctc aac ccg gtg gat gcc tat atg ctc tgc tcg gcc tgc      864
   Thr Gln Asn Leu Asn Pro Val Asp Ala Tyr Met Leu Cys Ser Ala Cys
           275                 280                 285
   ggt gat ctg cgt atc agc gaa atc gtc gat cag ccg aac tgg gtc gta      912
   Gly Asp Leu Arg Ile Ser Glu Ile Val Asp Gln Pro Asn Trp Val Val
       290                 295                 300
   tcg ttc tac ttc ccg cgt tcc gtt ttc gaa taa                          945
   Ser Phe Tyr Phe Pro Arg Ser Val Phe Glu
   305                 310
<210> 2
   <211> 314
   <212> PRT
   <213> Ochrobactrum anthropi
<400> 2
   Met Cys Asn Asn Cys His Tyr Thr Ile His Gly Arg His His His Phe
   1               5                   10                  15
   Gly Trp Asp Asn Ser Phe Gln Pro Ala Glu Thr Val Ala Pro Gly Ser
   20 25 30
   Thr Leu Lys Phe Glu Cys Leu Asp Ser Gly Ala Gly His Tyr His Arg
   35 40 45
   Gly Ser Thr Val Ala Asp Val Ser Thr Met Asp Phe Ser Lys Val Asn
   50 55 60
   Pro Val Thr Gly Pro Ile Phe Val Asp Gly Ala Lys Pro Gly Asp Val
   65 70 75 80
   Leu Lys Ile Thr Ile His Gln Phe Glu Pro Ser Gly Phe Gly Trp Thr
   85 90 95
   Ala Asn Ile Pro Gly Phe Gly Leu Leu Ala Asp Asp Phe Lys Glu Pro
   100 105 110
   Ala Leu Ala Leu Trp Asn Tyr Asn Pro Thr Thr Leu Glu Pro Ala Leu
   115 120 125
   Phe Gly Glu Arg Ala Arg Val Pro Leu Lys Pro Phe Ala Gly Thr Ile
   130 135 140
   Gly Val Ala Pro Ala Glu Lys Gly Leu His Ser Val Val Pro Pro Arg
   145 150 155 160
   Arg Val Gly Gly Asn Leu Asp Ile Arg Asp Leu Ala Ala Gly Thr Thr
   165 170 175
   Leu Tyr Leu Pro Ile Glu Val Glu Gly Ala Leu Phe Ser Ile Gly Asp
   180 185 190
   Thr His Ala Ala Gln Gly Asp Gly Glu Val Cys Gly Thr Ala Ile Glu
   195 200 205
   Ser Ala Met Asn Val Ala Leu Thr Leu Asp Leu Ile Lys Asp Thr Pro
   210 215 220
   Leu Lys Met Pro Arg Phe Thr Thr Pro Gly Pro Val Thr Arg His Leu
   225 230 235 240
   Asp Thr Lys Gly Tyr Glu Val Thr Thr Gly Ile Gly Ser Asp Leu Trp
                   245                 250                 255
   Glu Gly Ala Lys Ala Ala Leu Ser Asn Met Ile Asp Leu Leu Cys Gln
               260                 265                 270
   Thr Gln Asn Leu Asn Pro Val Asp Ala Tyr Met Leu Cys Ser Ala Cys
           275                 280                 285
   Gly Asp Leu Arg Ile Ser Glu Ile Val Asp Gln Pro Asn Trp Val Val
       290                 295                 300
   Ser Phe Tyr Phe Pro Arg Ser Val Phe Glu
   305                 310